# EUROPEAN PATENT APPLICATION

(11) **EP 1 557 172 A1**
(43) Date of publication of application: **27.07.2005**
(21) Application number: 03795258.7
(22) Date of filing: 29.08.2003
(51) Int. Cl.: A61K 38/00, A61K 38/21, A61K 38/36, A61K 38/53, A61K 48/00, A61P 35/00, A61P 37/02, A61P 37/06, A61P 37/08, A61P 43/00

(54) **METHOD AND COMPOSITION FOR REGULATING THE ACTIVITY OF REGULATORY T CELLS**

(30) Priority: 30.08.2002 JP 2002254967
(71) Applicant: ImmunoFrontier, Inc. a Japanese company, Mie 514-0061 (JP)
(72) Inventor: SHIKU, Hiroshi, Tsu-shi, Mie 514-0061 (JP)
(74) Representative: W.P. THOMPSON & CO.
(86) International application number: PCT/JP2003/011080
(87) International publication number: WO 2004/024174

(57) **Abstract**

The present invention provides a composition and method of regulating the activity of regulatory T cells. The present invention relates to a composition containing an antigen recognized by CD4⁺CD25⁺regulatory T cells or an expression vector encoding such an antigen and a method of controlling an immune response from a mammal by administrating the composition to the mammal. Furthermore, the present invention provides effective means for suppressing a rejection reaction and a graft-versus-host reaction in transplantation and for prevention and treatment of an autoimmune disease or an allergic disease. Furthermore, an immunosuppression condition can be removed by the administration of Interferon-γ or a combination of Interleukin 12 and Interleukin 18. In other words, those cytokine actions and the sensitization with an SEREX antigen are suitably combined to artificially manipulate regulatory T cells, allowing the cells to be applied to an autoimmune disease, reactions accompanied with organ transplantation, allergic reaction, control of tumor immunity, and the like.

## Description

### Industrial Field to which the Invention belongs

The present invention relates to a method of regulating the activity of regulatory T cells involved in regulation of immunity in the living body (*in vivo*), in particular a composition having activity for such regulation.

### Prior Art

Human beings have a biological defense system to remove exogenous materials and simultaneously have established self-tolerance. Such immune responses are induced and regulated by interactions among B-lymphocytes, T-lymphocytes, antibodies, and antigen-presenting cells (APCs). First, exogenous antigens are subjected to processing with the APCs and then bound to major histocompatibility complex (MHC) class-1 and class-2 molecules, followed by being presented to helper T cells. As the helper T cells recognize the exogenous antigens bound to MHCs, the T cells are activated, thereby secreting cytokines to assist the differentiation from B-cells stimulated by the antigens to antibody-producing cells and also to accelerate the differentiation of killer T cells. Cells presenting the antigen are eliminated by the secreted antibodies and the activated killer T cells. Thus, cellular and humoral reactions for removing the exogenous antigens progress. That is, the T cells play a central role and recognize antigens to be targeted, initiating an immune response. For example, in an antitumor immune response, it has been known for a long time that both CD4⁺ T cells and CD8⁺ T cells play an extremely important role (L. Gross, Cancer Res. 3: 326-333 (1943) ; L. Old et al., Ann. N. Y. Acad. Sci. 101: 80-106 (1962); R. J. North, Adv. Immunol. 35: 89 - 155 (1984); P. D. Greenberg, Adv. Immunol. 49: 281 - 355 (1991); D. M. Pardoll and S. L. Topalian, Curr. Opin. Immunol. 10: 588 - 594 (1998)). Even though CD8⁺CTLs (cytotoxic T cells) are major effector cells having ability to directly destroy tumor cells both *in vivo* and *in vitro,* they have been considered to be effecter cells that represent unique immune responses such that they are strict on the specificity of antigenic peptides bound to MHC class I, while natural killer T (NKT) cells are considered to be effector cells for intrinsic immune response with a moderate restriction of antigenic specificity. (M. J. Smyth et al., J. Exp. Med. 191: 661-668 (2000); M. J. Smyth &D. I. Godfray, Nature Immunol. 1: 459-460; M. J. Smyth et al., Curr. Opin. Immunol. 14: 165-171 (2002); T. Kawano et al., Proc. natl. Acad. Sci. USA 95: 5690-5693 (1998)). On the other hand, CD4⁺ T cells do not directly destroy tumor cells but they may play a basic role of controlling antitumor immune responses via various mechanisms (K. Hung et al., J. Exp. Med. 188: 2357-2368 (1998); F. Ossendorp et al., J. Exp. Med. 187 : 693-702 (1998) ; D. M. Pardoll & S. L. Toplian, Curr. Opin. Immunol. 10: 588-594 (1998) ; R. F. Wang, Trends. Immunol. 5: 269-276 (2001)). The CD4⁺ helper T cells that have recognized tumor antigenic peptides bound to MHC class II molecules amplify the activation and proliferation of CTLs by interaction with antigen-presenting cells (APCs). In contrast, CD4⁺CD25⁺ regulatory T cells (Treg) have been shown to be effective in preventing antitumor immune responses and the development of various autoimmune diseases (E. M. Schvach, Annu. Rev. Immunol. 18: 423-449 (2000); M. G. Roncarolo & M. K. Levings, Curr. Opin. Immunol. 12: 676-683 (2000) ; J. Shimizu et al, J. Immunol. 163: 5211-5218 (1999) ; S. Sakaguchi et al. , Immunol. Rev. 182: 18-32 (2001)). It, however, is not clearly shown what will be autoantigenic peptides recognized by Treg and what will be the difference of Treg from or relationship of Treg with helper T cells with respect to their antigenic recognition and functions. (S. Sakaguchi, Nature Immunol. 2: 283-284 (2001) ; K. J. Maloy & F. Powrie, Nature Immunol. 2: 816-822 (2001); E. M. Shevach, Nature Rev. Immunol. 6: 389-400 (2002)).

In the quantitative and qualitative amplification of CTLs, a possibility that the helper T cells implicated in antitumor immune responses recognize a wide range of various antigens has been suggested for successive cellular interactions among helper T cells, CTLs, and APCs (J. P. Ridge et al., Nature 393: 474-478 (1998); S. R. M. Bennett et al., Nature 393: 478-480 (1998); S. P. Schoenberger et al., Nature 393: 480-483 (1998); Z. Lu et al., J. Exp. Med. 191: 541-550 (2000)). The inventor of the present invention has found out that an extremely enhanced CTL activity and enhanced ability to cause a tumor rejection reaction can be induced by application of the SEREX (serological identification of antigen by recombinant cDNA expression cloning) antigen together with the CTL recognition antigen (H. Nishikawa et al., Proc. Natl. Acad. Sci. USA 98: 14571-14576 (2001); WO 03/000894 A1). Consequently, the inventor of the present invention has reached the invention with respect to vaccine for expressing tumor specific immunity where a composition is utilized as a polynucleotide vaccine, which includes expression vectors encoding a CD4⁺ helper antigen, preferably a molecule found by the SEREX method, and an antigen recognized by CD8⁺CTL, preferably a tumor antigen (WO 03/000894 A1), respectively.

The CD4⁺CD25⁺T regulatory cells are always produced in a functionally matured state from the normal thymus. However, those cells are typically in an anergic state, so that they will strongly suppress the activation and proliferation of other T cells with the introduction of T cell receptor (TCR) stimuli. On that occasion, it has been revealed that stimulus transduction through CTLA-4 (cytotoxic T lymphocyte-associated antigen 4) and GITR (glucocorticoid-induced tumor necrosis factor receptor family) molecules which are constitutively expressed is required for expression of suppression ability (T. Takahashi et al., J. Exp. Med. 192: 303-310 (2000); S. Read et al., J. Exp. Med. 192: 295-302 (2000); J. Shimizu et al., Nature Immunol. 3: 135-142 (2002)). Treg has been considered to be composed of cells having a wide range of specificity to recognize autoantigen bound to MHC class II. However, the antigens are still not identified and there is no knowledge at all about mechanism of enhancing the immunosuppression with Treg.

### DISCLOSURE OF THE INVENTION

Normal individuals such as mice and human beings possess their immune systems as biological defense systems against exogenous materials, so that they will not attack themselves. Such self-tolerance may be based on three mechanisms of eliminating autoreactive immune cells, inactivating autoreactive immune cells, and suppressing immunity with Treg. Then, autoimmune disease may occur when the self-tolerance fails, while the self-tolerance allows cancer cells to proliferate. In addition, for organ or tissue transplantation, a mechanism to eliminate exogenous antigens makes the transplantation difficult. Now that it becomes clear that Treg has an important role in immunosuppression in an immune system, it has been strongly expected to provide a method of regulating the activity of Treg and to develop a pharmaceutical agent against diseases due to immune disorder, especially autoimmune diseases, transplantation rejection reactions, graft-versus-host reactions, allergic diseases, and so on.

The inventor of the present invention has reached the present invention from his observation that the growth and metastasis of a tumor tissue have been more accelerated when an expression vector encoding SEREX antigen was administered alone instead of administering it together with an expression vector encoding tumor antigen to a cancer-bearing mouse to evaluate antitumor effects thereof.

The present invention provides a composition containing an antigen recognized by a CD4⁺CD25⁺ regulatory T cell. The present invention also provides a method of suppressing an immune response of a mammal, including administering the composition to the mammal. The present invention further provides a method of preventing and/or treating an autoimmune disease, including administering the composition to a mammal in a pharmaceutically effective dosage. Alternatively, the present invention provides a use of the composition, in which the composition is used in production of a preventive agent and/or a therapeutic agent for an autoimmune disease.

The present invention further provides a method of preventing and/or treating an allergic disease, including administering the composition to a mammal in a pharmaceutically effective dosage, or a use of the composition, in which the composition is used in production of a preventive agent and/or a therapeutic agent for an allergic disease.

The present invention also provides a method of suppressing a rejection reaction and/or a graft-versus-host reaction in an organ or tissue transplantation, including administering the composition to a mammal in a pharmaceutically effective dosage, or a use of the composition, in which the composition is used in production of a preventive agent and/or a therapeutic agent for suppressing a rejection reaction and/or a graft-versus-host reaction in an organ or tissue transplantation.

More specifically, there is provided a method of regulating the activity of regulatory T cells, where CD4⁺CD25⁺T cells (regulatory T cells) are sensitized by molecules (SEREX antigen) found by the SEREX (serological identification of antigen by recombinant cDNA expression cloning) method to enhance the activity of the regulatory T cells, while the activated regulatory T cells are reduced by the use of Interferon-γ or shared use of Interleukin 12 and Interleukin 18. In particular, a composition that enhances the activity of regulatory T cells may be a polynucleotide containing an expression vector encoding SEREX antigen. The polynucleotide may be administered alone into cells or may be fixed on carrier particles or the like and then administered into cells.

An object of the present invention is to realize the suppression of rejection and graft-versus-host reactions in transplantation, and the suppression of autoimmune diseases and allergic reactions by artificially sensitizing regulatory T cells with antigen to enhance immunosuppression activity, and to provide a therapeutic method effective for patients having those diseases or pathologic conditions.

The present invention relates to a method of immunosuppression on the basis of the finding that an immune reaction can be suppressed by administering an expression vector encoding antigen recognized by CD4⁺CD25⁺T cells. Therefore, the present invention relates to a composition containing an antigen recognized by CD4⁺CD25⁺T cells or an expression vector encoding such an antigen. More specifically, the antigen recognized by the CD4⁺CD25⁺T cells is preferably a molecule found by the SEREX method. In addition, the antigen may be a hetero antigen or an autoantigen, more preferably an autoantigen. The SEREX method is one developed in recent years by M. Pfreundschuh et al (Proc. Natl. Acad. Sci. USA94: 1914-1918 (1997)) and involves screening cDNA expression libraries of human tumors using human sera. In the Internet, more than 1,800 different types of genes identified by the SEREX method have been registered as SEREX database (www.licr.org/SEREX.html). They include, but not limited to, heat shock proteins DnaJ-like2 (GenBank Accession Nos.:NM_005494, XM_028966, XM_172161. XM_052862, XM_0627S4, XM_093388. NM_016306, NM_012328, and NM_005880), Galectin-8 (GenBank Accession Nos. :AH008815, AF193806, and AF193805), Poly(A)-binding protein (GenBank Accession No.: XM_067844), and Ligase 1 (GenBank Accession No.: NM_000234). The term "SEREX-identified molecules" as used herein refers to those molecules identified by the SEREX method.

In the present invention, polynucleotide that encodes an antigen may be DNA or RNA but not particularly limited as far as it can generate a desired immune response by administering the polynucleotide into a host animal according to the method of the present invention. The polynucleotide that encodes the antigen of the present invention may be any of those the nucleotide sequence of which is prepared by artificially deleting, substituting, inserting, and adding one or more amino acid sequences by means of well-known procedures such as a mutagenic method specific to a sequence site as far as a polypeptide encoded by the polynucleotide generates a desired immune response in the host (see, edit. Ausubel et al., Current Protocols in Molecular Biology (1987) John Wiley & Sons, Section 8.1-8.5). In addition, as far as a desired immune response is generated in a host animal, a variant found in the nature can be also isolated using a known hybridization technology (see, edit. Ausbel et al., Current Protocols in Molecular Biology (1987) John Wiley & Sons, Section 6.1-6.4) and a gene amplification technology (PCR) (see, edit. Ausbel et al., Current Protocols in Molecular Biology (1987) John Wiley & Sons, Section 6.1-6.4).

Furthermore, when a gene that encodes an antigen protein is known, a person skilled in the art will carry out without difficulty analysis of hydrophobic/hydrophilic regions on the amino acid sequence of such a protein (Kyte and Doolittle, J. Mol. Biol. 157: 105-122 (1982)), analysis of a secondary structure (Chou and Fasman, Ann. Rev. Biochem. 47: 251-276 (1978)), estimation of an antigenic region in the amino acid sequence (see, for example, Anal/Biochem. 151: 540-546 (1985)), and synthesis of a peptide corresponding to the estimated amino acid sequence to determine the antigenecity thereof according to the PEPSCAN method (Nature 314 (1985) and JP 60-500684 A) or the like. Therefore, a polynucleotide that encodes a peptide fragment containing an epitope portion defined by the above method can be produced by any of procedures including a chemical synthesis and can be also used as an expression vector for the antigen of the present invention.

The expression vector used in the present invention is a recombinant vector with the insertion of an antigen gene recognized by CD4⁺CD25⁺ regulatory T cells. Examples of the vector to which an antigen gene is inserted include plasmids, phages, cosmids, viruses, and other vectors conventionally used in the art. Any person skilled in the art will construct various kinds of plasmids and vectors using technologies described in, for example, Sambrook et al., (Ed.), Molecular Cloning A Laboratory Manual, Cold Spring Harbor Laboratory (1989) N. Y.) and Ausubel et al. (Ed.), Current Protocols in Molecular Biology (1987) John Wiley & Sons.

Persons skilled in the art may suitably select factors for control of expression of antigen genes in host cells, such as promoters and terminators, from known control sequences appropriately and arrange them on the upstream or downstream of the genes on the basis of the types of the host cells and purposes, respectively. Therefore, the control sequence used may be originated from the antigen or a heterologous one. If required, a marker such as an antibiotic resistance marker may be used in the expression vector of the present invention. Although many commercially available vectors can be used, preferably those from which polynucleotide sequences unessential for the present invention are removed may be used.

The composition of the present invention may be used as a naked plasmid. That is, the composition of the present invention may be used in the form of being packed in a ribosome or of one of various kinds of virus vectors including a retrovirus vector, an adenovirus vector, a vaccinia virus vector, a pox virus vector, an adenovirus-associated vector, and an HVJ vector (see, e.g., K. Adolph "Virus Genome Method", CRC Press, Florida (1996)), or may be used in the form of being coated on beads (carriers) such as colloidal gold particles. The composition of the present invention preferably has, but not limited to, a configuration such that a vector for the expression of antigen recognized by CD4⁺CD25⁺ regulatory T cells is attached on carrier particles such as gold particles to be introduced into the living body by means of a gene gun or the like. The technologies for coating carrier particles with polynucleotides are known in the art (see, e.g. , WO 93/17706) . Finally, the polynucleotide can be prepared in a solution of saline or the like suitable for administration to the living body. Other additional agents such as calcium ions may be used together to promote the intracellular incorporation of plasmid. Furthermore, if required, other pharmaceutically acceptable agents that promote the transfection may be used together.

The composition of the present invention can be administered by any of administration methods. Preferably, however, the composition of the present invention is administered by means of injection, infusion, or gas-inductive particle-bombardment method (by an electron gun or the like) through a suitable parenteral route such as an intravenous, intraabdominal, subcutaneous, or intracutaneous route, an intra-adipose tissue or intra-breast tissue route, or an inhalation or intramuscular route; collunarium or the like through a mucosal route; or the like. Of those administration methods, genetic transformation techniques with accelerated particles are described in U.S. Patent Nos. 4, 945, 050 and 5,240,842 and devices based on the modified methods thereof have been commercially available (Biorad Laboratories).

The host animal species used in the present invention are not limited. However, specific examples of the species include mammals including mice, rats, rabbits, dogs, cats, pigs, sheep, caws, horses, and primates such as monkeys and human beings. Of those, for the host animals, human beings are more preferable.

### DETAILED DESCRPTION OF THE INVENTION

The antigen or expression vector used in the present invention may be generally administered at an amount of 0.001 µg to 1 g, preferably 0.01 *µ*g to 10 mg, more preferably 0.1 *µ*g to 100 *µ*g per dosage even though the dosage may vary depending on the type or degree of disease, the sexuality, age, and body weight of the animal, an administrating route, and so on, and is not limited.

The immunosuppression state realized as a result of carrying out the above process can be removed by administration of Interferon-γ or administration of Interleukin 12 together with Interleukin 18. In other words, an appropriate combination of the action of cytokine and the sensitization with SEREX antigen allows regulatory T cells to be artificially modified and applied to an autoimmune disease, a reaction with organ transplantation, allergic reaction, and control of tumor immunity.

Alternatively, furthermore, removal of an immunosuppression state may be carried out by the treatment with anti-CD4 antibody or anti-CD25 antibody.

Therefore, the present invention provides a method and composition for controlling the activity of regulatory T cells.

Since the present invention has found that SEREX antigen promotes the immunosuppression activity of regulatory T cells, there is shown a method of controlling the activity of regulatory T cells for the first time.

Conventionally, even though various procedures and agents have been developed for the treatments of autoimmune diseases and allergy as well as the treatments for suppressing transplantation rejection and graft-versus-host reaction, the effectiveness thereof reaches a limit. Therefore, those having higher effectiveness have been needed.

The present invention provides a method and composition extremely effective in controlling the action of CD4⁺CD25⁺ regulatory T cells the importance of which in the living body has been clarified more and more in late years, bringing a breakthrough in the treatments with therapy of autoimmune diseases and allergic diseases and organ and tissue transplantation.

### BRIEF DESCRIPTION OF THE DRAWINGS

Fig. 1 is a graph showing that the immunization with SEREX antigen alone promotes metastases to lung.
Fig. 2 is a graph showing that cells responsible for promoting metastases to lung induced by the immunization with SEREX antigen alone are CD4⁺ or CD4⁺CD25⁺ cells but not CD8⁺ cells.
Fig. 3 is a graph showing that transplantation of CD4⁺CD25⁺ T cells from the mouse immunized by SEREX antigen alone promotes metastases to lung.
Fig. 4 is a graph showing that the pre-treatment with SEREX antigen alone reduces the number of CD8⁺ T cells specific to mERK2 antigen.
   In the figure, 9m-pulsed P1HTR denotes mERK2 9m antigen protein and p63-71(T)-pulsed P1HTR denotes the control antigen protein.
Fig. 5 is a graph showing in vivo preventive and therapeutic effects of immunization with mERK2 or 147HER2 and Dna J-like-2 on metastases to lung.
Figs. 6, 7, 8, and 9 show results of measuring the diameters of tumors on the backs of the mice in Example 4.

### Examples

Hereinafter, the present invention will be described in more detail with reference to examples thereof. However, these examples have no intention of restricting the scope of the present invention in any sense.

### Reference Example: Inhibition of Metastases to Lung by Simultaneous Sensitization with SEREX Antigen and Tumor Antigen

*In vivo* injection of 3-methylcholanthrene-induced sarcoma CMS5m cells originated from BALB/c into animals causes metastases to lung, so that the animals will be dead within 5 to 6 weeks. Thus, a model of metastases to lung was established by administrating 1 × 10⁶ CMS5m tumor cells of 0.1 ml in total into a BALB/c mouse through the lateral caudal vein thereof. mERK2 which is a mutated kinase was used as a tumor antigen, and DnaJ-like2 was used as an SEREX antigen. Immunization was initiated every other weak such that the immunization was initiated before 14 days or 7 days from the tumor administration with 1) a plasmid encoding mERK2, 2) a mixture of the mERK2 plasmid and a plasmid encoding a control vector, and 3) a mixture of the mERK2 plasmid and a DnaJ-like2 plasmid, or on the day of the tumor administration (on the same date), or after 5 days from the tumor administration (according to the method described in H. Nishikawa et al., Proc. Natl. Acad. Sci. USA 98: 14571-14576 (2001)). After 28 days from the tumor administration, the mice were sacrificed and the number of pulmonary nodules was counted under dissecting microscopy. For each group, five animals were used. The results are shown by means ± SEM thereof.

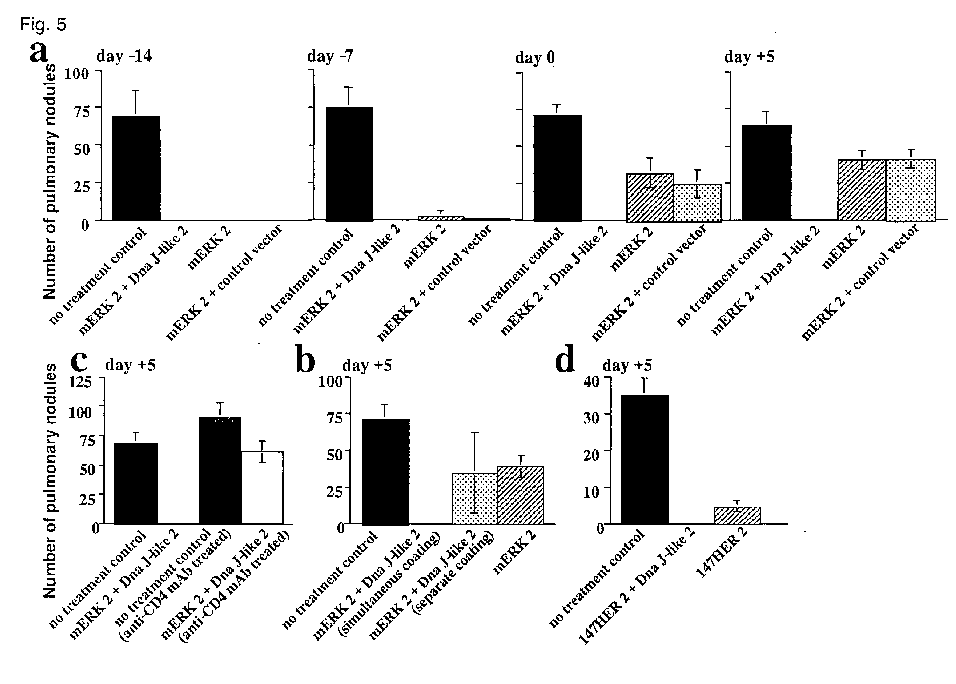

Before 14 days from the tumor administration, mice were immunized by plasmid encoding mERK2, resulting in complete prevention of metastases to lung. Such a preventive effect could not be observed when the immunization was carried out before 7 days from the tumor administration and also after the tumor administration (see Fig. 5). In contrast, the immunization with a combination of mERK2 plasmid and DnaJ-like2 plasmid prevented metastases to lung completely even after up to 5 days from the tumor administration (see Fig. 5). The presence or absence of metastases was confirmed by means of a histopathological examination. The numeral value in the figure shows means ± SEM of five mice for each group.

### Example 1: Promotion of Metastases to Lung by Sensitization with SEREX Antigen Alone

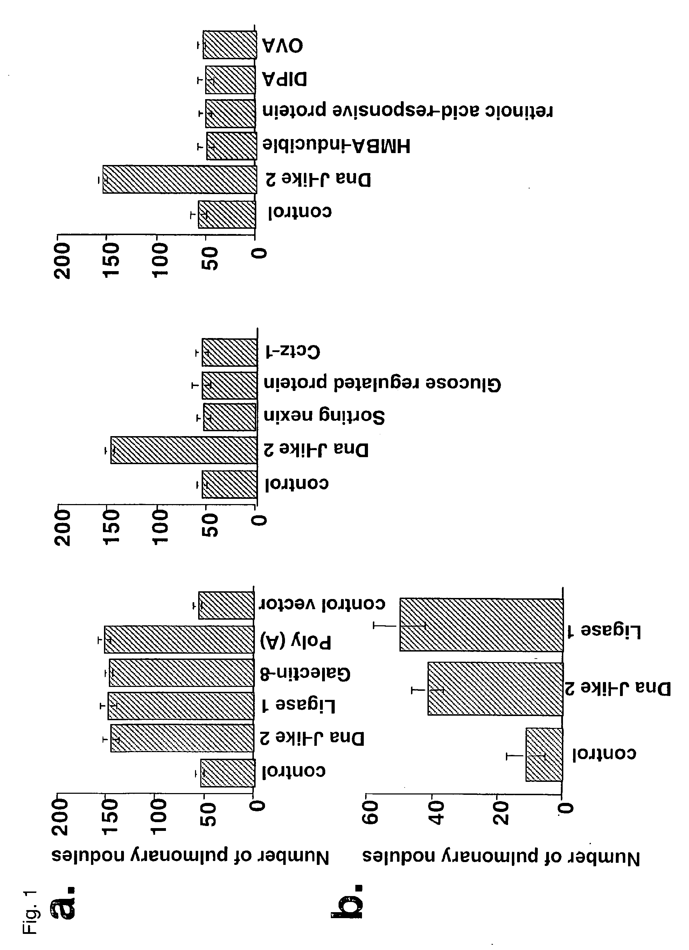

In the same model of metastases to lung as that of Reference Example, mice immunized by the plasmids encoding SEREX antigen or the control vectors after 5 days from the tumor administration were sacrificed after 28 days from the tumor administration, respectively. Then, the number of pulmonary nodules was counted for each animal. When the animals were immunized by the respective plasmids of four different antigens (each of them was a mouse's protein), i.e., a heat shock protein DnaJ-like2, DNA ligase 1, Galectin-8, and poly(A)-binding protein cytoplasmic, as the SEREX antigen, the number of the nodules was 130 to 170. In the case of no immunization like that, the number of the nodules was 30 to 50. On the other hand, when the animals were respectively immunized by plasmids encoding three mouse's molecules which could not be detected even after repeated SEREX analysis, i.e., glucose regulated protein, secreted nexin, and TCP-1 zeta subunit (Cctz-1) -containing chaperon, no metastasis promotion was observed (Figs. 1a and 1b). In addition, even if the animals were respectively immunized by plasmids encoding three human SEREX antigens (Homo sapiens HMBA-inducible protein, human retinoic acid response protein, and Homo sapiens hepatitis delta antigen-responsive protein A), and different species of proteins such as ovalbumin, no promotion of metastasis was observed (Fig. 1a). In other words, it was suggested that the sensitization with the SEREX antigen alone as autoantigen promoted metastases.

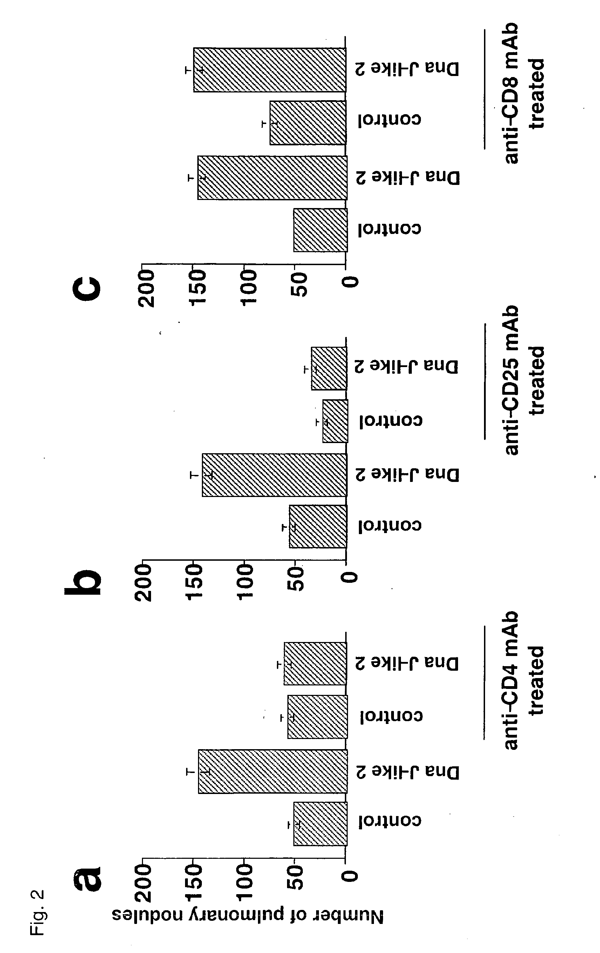

Therefore, cell phenotypes involved in the metastatic promotion were analyzed. When the mouse was pre-treated with anti-CD4 monoclonal antibody or anti-CD25 monoclonal antibody, no metastatic promotion due to the sensitization with DnaJ-like2 was observed at all (Figs. 2a and 2b). When the mouse was pre-treated with anti-CD25 monoclonal antibody, the number of metastatic nodules considerably decreased, compared with one which was not pre-treated (Fig. 2b). In addition, when the mouse was pre-treated with anti-CD8 monoclonal antibody and sensitized with DnaJ-like2, the metastatic promotion was observed as much as one observed in the case of no antibody pre-treatment (Fig. 2c). This result showed that CD4⁺ or CD4⁺CD25⁺ T cells were responsible for the metastatic promotion.

### Example 2: CD4⁺CD25⁺ T cells Sensitized by SEREX Antigen Promote Metastases to Lung

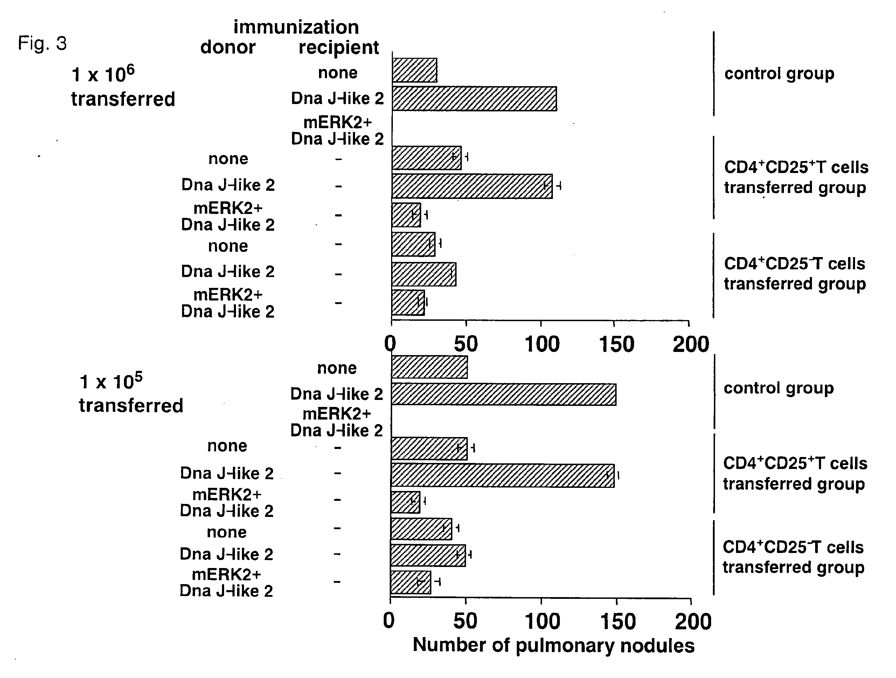

For directly validating the results obtained in Example 1, CD4⁺CD25⁺ T cells sensitized by SEREX antigen was transplanted in a tumor-inoculated mouse and the presence or absence of metastatic promotion were observed. That is, when CD4⁺CD25⁺ T cells were transplanted from a mouse immunized by DnaJ-like2 antigen into a tumor mouse, metastases to lung was considerably promoted. In contrast, when CD4⁺CD25⁻ T cells were transplanted, nearly no promotion of metastases to lung was observed. In addition, significant metastatic promotion was observed when unsensitized CD4⁺CD25⁺ T cells were transplanted, compared with no transplantation. In this case, however, the degree of metastatic promotion was extremely small, compared with the case in which sensitized CD4⁺CD25⁺ T cells were transplanted (Fig. 3).

### Example 3: Pre-treatment with SEREX Antigen Suppresses the Activity of CD4⁺ T cells but not CD8⁺ T cells

The inventor et al. of the present invention have previously reported that the administration of each of plasmids encoding tumor antigen and SEREX antigen to the tumor-inoculated mouse could induce a strong antitumor immune response, while considerably enhancing the helper function of CD4⁺ T cells, and besides the amplification of CD8⁺ T cell response was depended on CD4⁺ T cells (H. Nishikawa et al., Proc. Natl. Acad. Sci. USA 98: 14571-14576 (2001); WO 03/000894 A1).

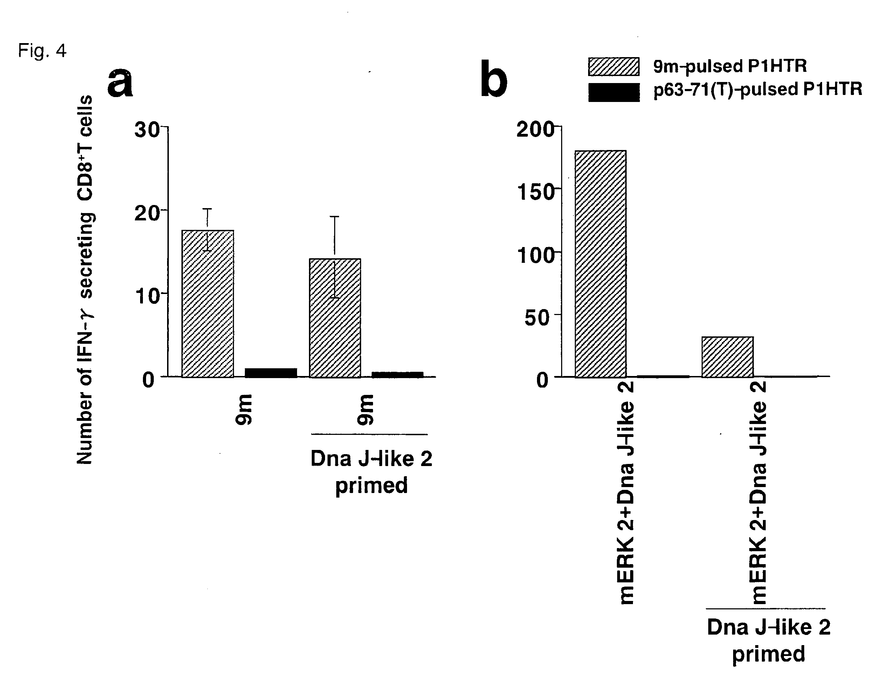

In view of the above, it was studied whether or not the immune responses of CD8⁺ T cells and CD4⁺ T cells changed with respect to the suppression on an antitumor immune response confirmed in Example 2. A 9-mer of mutated kinase (i.e., a peptide mERK2 9m) was used as a tumor antigen. A mouse was pre-immunized with DnaJ-like2 and then immunized with a plasmid encoding mERK2 9m alone or together with a plasmid encoding DnaJ-like2, followed by analyzing mERK2 9m specific CDS⁺T cells by the ELISPOT assay. If mERK2 9m was used alone, the pre-treatment with DnaJ-like2 did not affect CD8⁺ T cells (Fig. 4a). However, in the case of combined immunization, enhanced helper activity observed at the time of no pre-treatment with DnaJ-like2 was completely disappeared by the pre-treatment with DnaJ-like2 (Fig. 4b). Consequently, it was found that the immunization with SEREX antigen suppressed the helper activity of CD4⁺T cells but not CDS⁺T cells. The analysis with α-Galcer-CD1d tetramer confirmed that the number of local natural killer T (NKT) cells decreased.

From Example 1, Example 2, and Example 3 described above, as an example of the antitumor immune response of a mouse tumor metastasis model, it is experimentally proved that the treatment with a plasmid encoding the SEREX antigen alone suppresses the entire immune response by suppressing the activity of CD4⁺ helper T cells as a result of the activation of the CD4⁺CD25⁺ regulatory T cells.

### Example 4: Control of Alloimmune Response by Control of Activity of Regulatory T cells

The inventor of the present invention has identified a wild-type autoantigen without mutation recognized by the immune system of a cancer-bearing individual by means of the SEREX method using a cancer-bearing mouse serum (SEREX-identified autoantigen). The SEREX-identified autoantigen may be recognized by CD4 cells after being recognized by IgG. An enhanced helper effect can be exerted when those SEREX-identified autoantigen and cytotoxic T cell (CTL) recognizing cancer rejection antigen are used for co-immunization using a Helios-type gene gun (Gene Gun^{R}, Helios, Co., Ltd.).

On the other hand, for the immunization with SEREX-identified autoantigen alone, it is found that the tumor in a lung metastasis model of a murine sarcoid becomes worse and the immunity is regulated suppressively.

This mechanism is considered to be used for the immunization of sole SEREX-identified autoantigen with a decrease in NKT by activating regulatory CD4⁺CD25⁺T cells. In this example, for investigating how an immunosuppression effect due to the immunization with sole SEREX-identified autoantigen participates in an alloimmune response in transplantation or the like, the tumor originated from a mouse of another strain was inoculated into a mouse immunized by the SEREX-identified autoantigen alone, and the effect of the inoculation on proliferation was investigated.

### (1) Materials and Methods

Mice: BALB/c(H-2d) and C57BL/6(H-2b) mice were used.
Plasmids: SEREX-identified autoantigen genes (DnaJ-like2, Mus DNA Ligase 1 (Ligase 1), Mus poly(A)-binding protein, and cytoplasmic 1 (Poly (A)) were incorporated into the respective pBK-CMV plasmids and then coated on gold particles.
Gene Introduction (Immunization): Immunization with the SEREX-identified autoantigen alone was carried out by intracutaneously supplying into the abdomen of a mouse by using the Helios-type gene gun.
Tumor Inoculation: After one week from the second immunization with the SEREX-identified autoantigen alone, a BALB/c mouse was inoculated with a melanoma cell line B16 originated from C57BL/6 and a C57BL/6 mouse was inoculated with a sarcoma cell line CMS5a originated from BALB/c, which were subcutaneously inoculated in the backs thereof at a concentration of 1 × 10⁶.

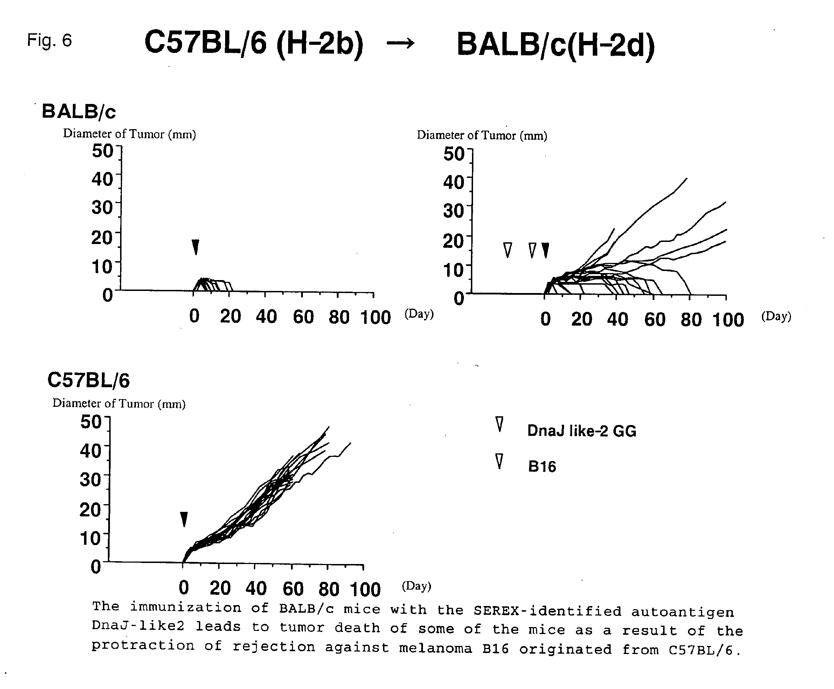

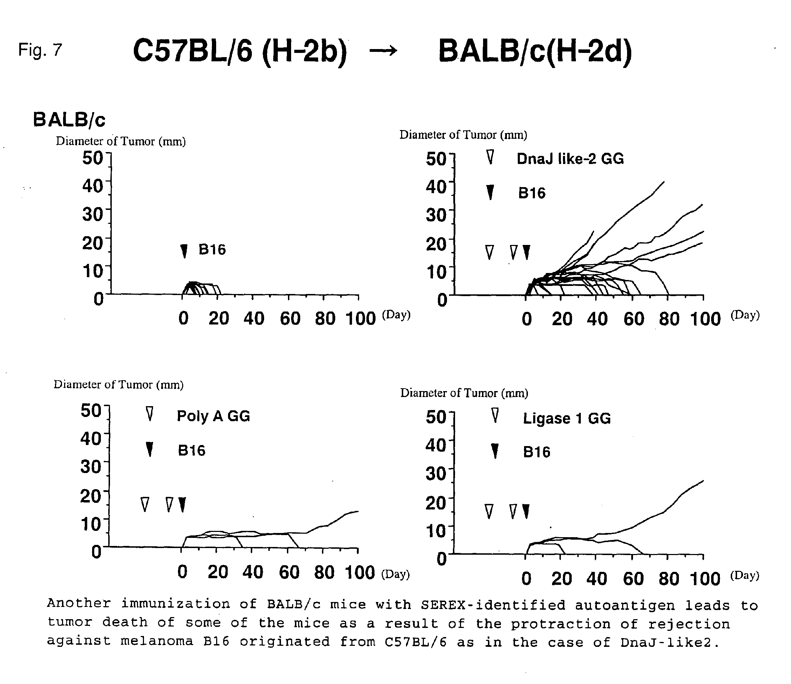

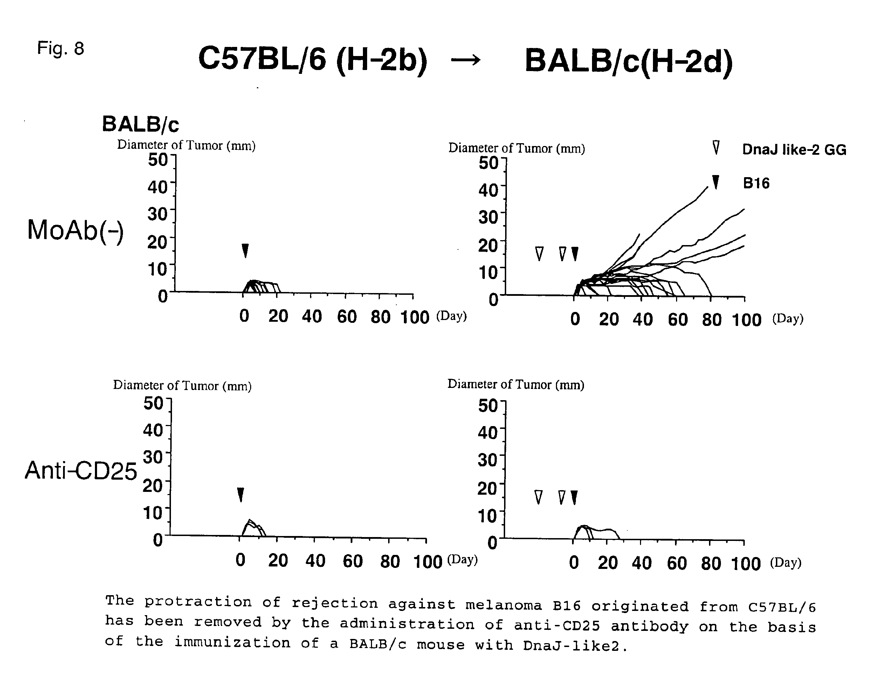

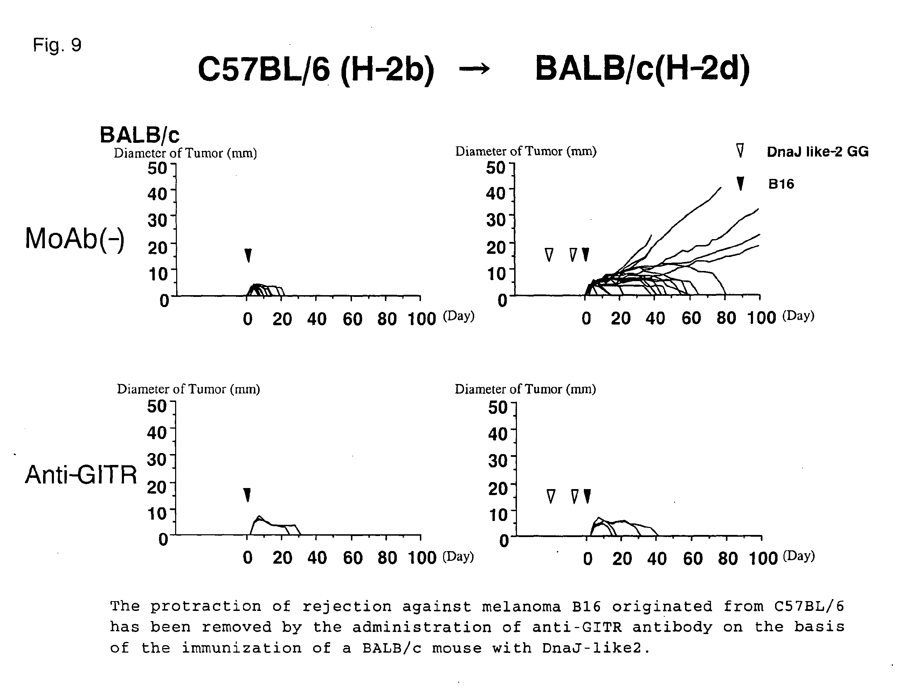

After tumor inoculation, the diameter of the tumor of the mouse's back was measured over time (See Figs. 6 to 9).

Antibodies: An anti-CD25 antibody was administered at a concentration of 0.25 mg after concentration and fractionation of the antibody with ammonium sulfate from hybridoma PC61.

As the anti-GITR (Glucocorticoid-induced TNF receptor superfamily 18) antibody, a hybridoma DTA-1 (Prof.ShimonSakaguchi, Kyoto Univ.) peritoneal fluid was diluted 8 times and then 200µl (25 µl) thereof was provided. As the anti-CD4 antibody, a hybridoma GK1.5 peritoneal fluid was diluted 8 times and then 200 µl (25 µl) thereof was provided. For the respective antibodies, the administration of antibody was performed once immediately before the inoculation of tumor.

### (2) Results and Discussion

For BALB/c mice, rejection of the tumor originated from C57BL/6 mice was prolonged by immunization of SEREX-identified autoantigen and some of the mice were dead of tumor.

This phenomenon was cancelled by the administration of anti-CD25 antibody or the administration of anti-GITR antibody. Thus, the CD4⁺CD25⁺ T cells may participate.

As a result of suppressive regulation of the immune response by the immunization of SEREX-identified autoantigen, an alloimmune response may have also been reduced.

## Claims

1. A composition comprising an antigen recognized by a CD4⁺CD25⁺ regulatory T cell.

2. The composition according to claim 1, wherein the antigen recognized by the CD4⁺CD25⁺ regulatory T cell comprises a molecule identified by an SEREX method.

3. A composition comprising an expression vector that encodes an antigen recognized by a CD4⁺CD25⁺ regulatory T cell.

4. The composition according to claim 3, wherein the antigen recognized by the CD4⁺CD25⁺ regulatory T cell comprises a molecule identified by the SEREX method.

5. The composition according to claim 2 or claim 4, wherein the molecule identified by the SEREX method comprises an autoantigen.

6. The composition according to any one of claims 2, 4, and 5, wherein the molecule identified by the SEREX method comprises one selected from the group consisting of DnaJ-like2 (GenBank Accession No.:NM_005494, XM_028966, XM_172161, XM_052862, XM_062754, XM_093388, NM_016306, NM_012328, and NM_005880), Galectin-8 (GenBank Accession No.:AH008815, AF193806, and AF193805), poly(A)-binding protein (GenBank Accession No. :XM_067844), and Ligase-1 (GenBank Accession No.:NM_000234).

7. The composition according to any one of claims 3 to 6, wherein a gene gun is used for administration of the composition.

8. The composition according to any one of claims 1 to 7, wherein the composition is provided for preventing and/or treating autoimmune diseases.

9. The composition according to any one of claims 1 to 8, wherein the composition is provided for preventing and/or treating allergic diseases.

10. The composition according to any one of claims 1 to 9, wherein the composition is intended for suppressing a rejection reaction and/or a graft-versus-host reaction in an organ or tissue transplantation.

11. A method of immunosuppressing a mammal, comprising administering the composition according to any one of claims 1 to 10 to the mammal.

12. A method of immunosuppressing a mammal, comprising the steps of administering the composition according to any one of claims 1 to 10 to the mammal to suppress an immune response; and further administering Interferon-γ to recover the immune response from the mammal.

13. A method of immunosuppressing a mammal, comprising the steps of administering the composition according to any one of claims 1 to 10 to the mammal to suppress an immune response; and further administering Interleukin 12 and Interleukin 18 to recover the immune response from the mammal.

14. A method of preventing and/or treating an autoimmune disease, comprising administering the composition according to claim 1 to a mammal in a pharmaceutically effective dosage.

15. Use of the composition according to any one of claims 1 to 10 for manufacturing a preventive agent and/or a therapeutic agent for an autoimmune disease.

16. A method of preventing and/or treating an allergic disease, comprising administering the composition according to claim 1 to a mammal in a pharmaceutically effective dosage.

17. A use of the composition according to any one of claims 1 to 10, wherein the composition is used in production of a preventive agent and/or a therapeutic agent for an allergic disease.

18. A method of suppressing a rejection reaction and/or a graft-versus-host reaction in an organ or tissue transplantation, comprising administering the composition according to claim 1 to a mammal in a pharmaceutically effective dosage.

19. Use of the composition according to any one of claims 1 to 10 for manufacturing a preventive agent and/or a therapeutic agent for suppressing a rejection reaction and/or a graft-versus-host reaction in an organ or tissue transplantation.
